# EUROPEAN PATENT SPECIFICATION

(11) **EP 0 571 434 B1**
(45) Date of publication and mention of the grant of the patent: **02.08.1995**
(21) Application number: 92904133.3
(22) Date of filing: 10.02.1992
(51) Int. Cl.: C07D 487/04, A61K 31/40

(54) **A PROCESS FOR THE PREPARATION OF 1,3a,8-TRIMETHYL-1,2,3,3a,8,8a-HEXAHYDRO-PYRROLE 2,3-b]INDOL-5(3aS,8aR)-HEPTYLCARBAMATE**
Verfahren zur Herstellung von 1,3A,8-Trimethyl-1,2,3,3A,8,8A-HEXAHYDROPYROLO(2,3-B)INDOL-5(3AS,8AR)-HEPTYLCARBAMAT
PROCEDE DE PREPARATION DE 1,3a,8-TRIMETHYLE-1,2,3,3a,8,8,8a-HEXAHYDRO-PYRROLE 2,3-b]INDOLE-5(3aS,8aR)-HEPTYLCARBAMATE

(30) Priority: 14.02.1991 IT MI910388
(43) Date of publication of application: 01.12.1993
(73) Proprietor: LABORATORIO CHIMICO INTERNAZIONALE S.p.A., 20122 Milano (IT)
(72) Inventor: MAIORANA, Stefano, I-20122 Milano (IT); BONURA, Anna, I-20122 Milano (IT); CHIODINI, Giorgio, I-20122 Milano (IT)
(74) Representative: Bianchetti, Giuseppe
(86) International application number: EP9200278
(87) International publication number: WO9214735

(56) References cited:
- EP-A- 0 298 202

## Description

The present invention relates to a process for the preparation of 1,3a,8-trimethyl-1,2,3,3a,8,8a-hexahydropyrrole[2,3-b]indol-5(3aS,8aR)-heptylcarbamate of formula (I): 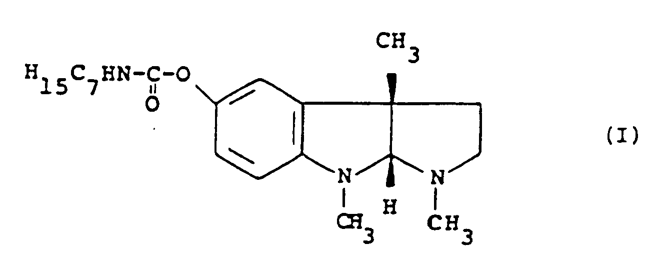
Said compound, which is known under the International Nonproprietary Name "heptastigmine", and methods for the preparation thereof, are disclosed in EP-A-154864 and EP-A-298202. The compound has interesting pharmacological properties making it useful particularly in the treatment of Alzheimer's disease.

According to the above cited Patent Applications, heptastigmine (I) can be obtained starting from eseroline (II) 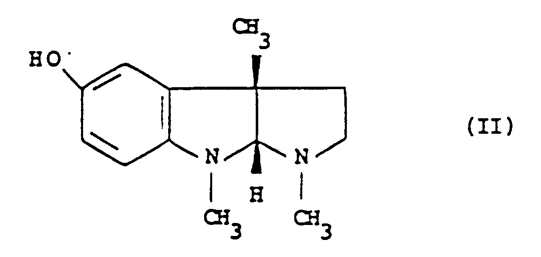
by treatment either with phosgene and heptylamine, or with heptylisocyanate. The latter method allows to obtain higher yields than the first one, as far as the reaction is carried out in an ether solution and in the presence of metal sodium.

Said methods, however, suffer form evident drawbacks, particularly on industrial scale. In fact, the first method involves the use of highly toxic phosgene, whereas the second one suffers from hazards of fires and explosions, connected to the use of ether and metal sodium.

Now, it has surprisingly been found that heptastigimine (I) can be obtained in very good yields and high purity, by reacting eseroline (II) with heptylisocyanate in the presence of a) catalytic amounts of a quaternary ammonium salt and b) catalytic amounts of a metal cyanate, in a polar aprotic solvent, according to the scheme reported hereinbelow: 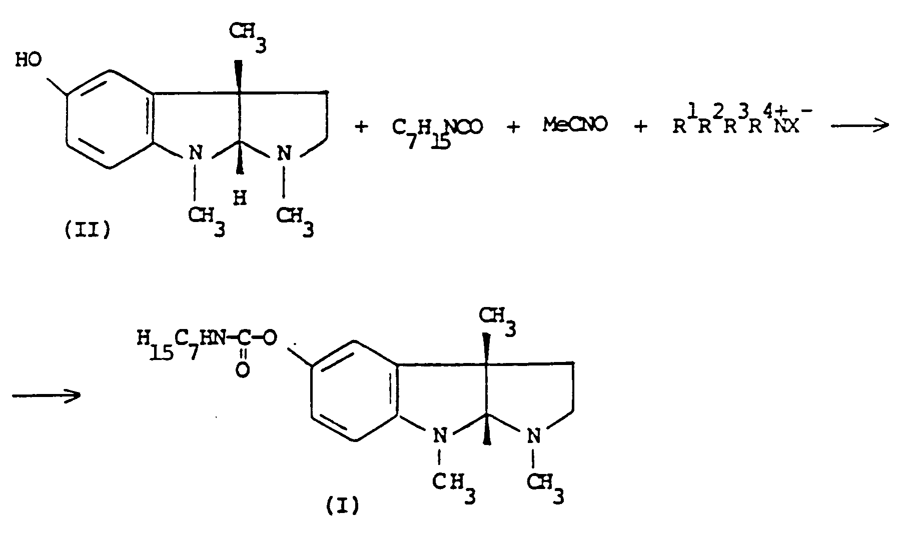
wherein Me is an alkali metal or the equivalent of an alkaline-earth metal, and preferably potassium; R₁-R₄, which can be the same or different, are C₁-C₁₂ alkyl or aralkyl, preferably ethyl or n-butyl; X is an anion, preferably chlorine or bromine.

The molar ratio of eseroline to heptylisocyanate is comprised between 1:1 and 1:5, preferably between 1:1 and 1:1.2. Heptylisocyanate amounts which are in excess or defect with respect to the preferred range can also be used, but no advantages are obtained.

The molar ratio of eseroline to metal cyanate can range between 1:0.5 and 1:0.01, preferably between 1:0.4 and 1:0.1, whereas the molar ratio of eseroline to quaternary ammonium salt is comprised between 1:0.6 and 1:0.05, preferably between 1:0.5 and 1:0.2.

Acetonitrile and ethyl acetate are preferably used as the polar aprotic solvent.

It has also been found that metal alcoholates, particularly potassium t-butylate, may substitute in some cases the metal cyanate.

The reaction is preferably carried out under nitrogen, in order to prevent eseroline oxidation, and at temperatures comprised between 0°C and 40°C, preferably at about 20°C. The order in which reagents are added is not particularly critic; however heptylisocyanate, optionally diluted with the solvent, is preferably added to a solution or suspension of eseroline, metal cyanate and quaternary ammonium salt in the solvent itself. At the end of the reaction (the progress of which can be followed with chromatographic methods, for example by TLC mix, eluent CH₂Cl₂/MeOH 9:1) solvent is evaporated under reduced pressure, the residue is taken up with toluene, the unsolubles are filtered off and the toluene solution is repeatedly washed with water. Toluene is evaporated off to obtain the desired product as a viscous oil, which is left to crystallize or is transformed into the corresponding tartrate, according to indications of Italian Patent Application N. 19964 A/87.

According to an alternative route, eseroline can be N-heptylcarbamoylated with heptylisocyanate which is prepared in situ starting from a 1-haloheptane (preferably 1-bromoheptane) and a metal cyanate (preferably potassium cyanate) in the presence of a quaternary ammonium salt, in polar aprotic solvents, preferably acetonitrile. In this case the reaction is carried out (always under nitrogen protection) at the reflux temperature of the mixture; compound (I) is then recovered as described above.

The following Examples further illustrate the invention.

### EXAMPLE 1

500 g (2.3 moles) of eseroline are dissolved in 20 l of anhydrous acetonitrile, under nitrogen; 37 g (0.46 mole) of potassium cyanate and 148 g (0.69 mole) of tetraethylammonium bromide are added thereto and, at 19-20°C, a solution of 325 g (2.5 moles) of heptylisocyanate in 20 l of anhydrous acetonitrile is quickly dropped therein. The reaction mixture is kept under stirring for 8 hours, then the insolubles are filtered off, solvent is evaporated off under reduced pressure, the residue is taken up into 100 l of toluene and the toluene solution is washed with 6 x 100 l of water.

Toluene is evaporated under vacuum to yield a yellowish viscous oil which undergoes solidification and can be transformed into the corresponding tartrate according to the indications of Italian Patent Application n. 19964 A/87. Yield 90%.

### EXAMPLE 2

150 g of eseroline (0.69 mole), 83.9 g of potassium cyanate (1.035 moles), 148 g of 1-bromoheptane (0.83 mole) and 29 g of tetraethylammonium bromide (0.14 mole) are placed into 5 l of anhydrous acetonitrile. The reaction mixture is refluxed for 19 hours, the insolubles are filtered off and the mixture is worked up as described in Example 1. Heptastigmine yield is 63%.

### EXAMPLE 3

The procedure of Example 1 is followed, but replacing tetraethylammonium bromide with an equivalent amount of tetra-n-butylammonium bromide. The yield is still 90%.

### EXAMPLE 4

The same results as those of Example 2 are obtained using n.Bu₄NBr⁻ instead of (C₂H₅)₄NBr⁻, the other reaction conditions being unchanged.

## Claims

1. A process for the preparation of 1,3a,8-trimethyl-1,2,3,3a,8,8a-hexahydro-pyrrole[2,3-b]indol-5(3aS,8aR)-heptylcarbamate of formula (I): 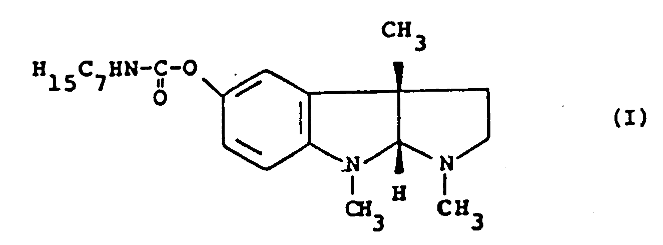 comprising the reaction of eseroline (II) with heptylisocyanate characterized in that said reaction is carried out in the presence of a) catalytic amounts of a quaternary ammonium salt and b) catalytic amounts of a metal cyanate, in a polar aprotic solvent, according to the scheme reported hereinbelow: 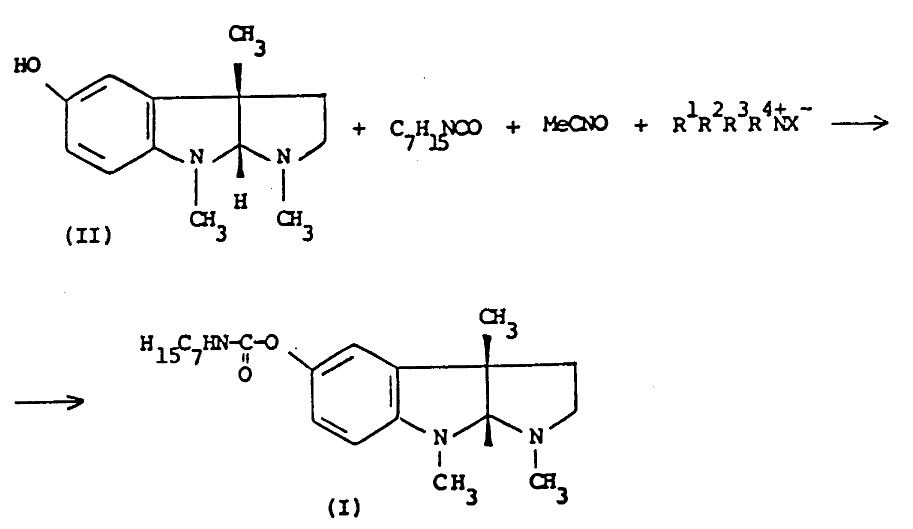 wherein Me is an alkali metal or the equivalent of an alkaline-earth metal; R₁-R₄, which can be the same or different, are C₁-C₁₂ alkyl or aralkyl; X is an anion.

2. A process according to claim 1, characterized in that the molar ratio of eseroline to heptylisocyanate is comprised between 1:1 and 1:5.

3. A process according to claim 1, characterized in that the molar ratio of eseroline to heptylisocyanate is comprised between 1:1 and 1:1.2.

4. A process according to claims 1 to 3, characterized in that the molar ratio of eseroline to metal cyanate is comprised between 1:0.5 and 1:0.01.

5. A process according to claims 1 to 3, characterized in that the molar ratio of eseroline to metal cyanate is comprised between 1:0.4 and 1:0.1.

6. A process according to any one of the previous claims, characterized in that the molar ratio of eseroline to quaternary ammonium salt is comprised between 1:0.6 and 1:0.05.

7. A process according to any one of the previous claims, characterized in that the molar ratio of eseroline to quaternary ammonium salt is comprised between 1:0.5 and 1:0.2.

8. A process according to any one of the previous claims, characterized in that acetonitrile is used as the solvent.

9. A process according to any one of the previous claims, characterized in that the reaction is carried out at temperatures comprised between 0°C and 40°C.

10. A process according to any one of claims 1-8, characterized in that eseroline is reacted with heptylisocyanate which is prepared in situ starting from a 1-haloheptane and a metal cyanate in the presence of a quaternary ammonium salt, in polar aprotic solvents.

11. A process according to claim 1, wherein Me is potassium; C₁-C₁₂ alkyl is ethyl or n-butyl; X is chlorine or bromine.

12. A process according to claim 10, wherein the 1-haloheptane is 1-bromoheptane and the metal cyanate is potassium cyanate.

13. A process according to claim 10, wherein the polar aprotic solvent is acetonitrile.

## Patentansprüche

1. Verfahren zur Herstellung von 1,3a,8-Trimethyl-1,2,3,3a,8,8a-hexahydro-pyrrol[2,3-b]indol-5(3aS,8aR)-heptylcarbamat der Formel (I): 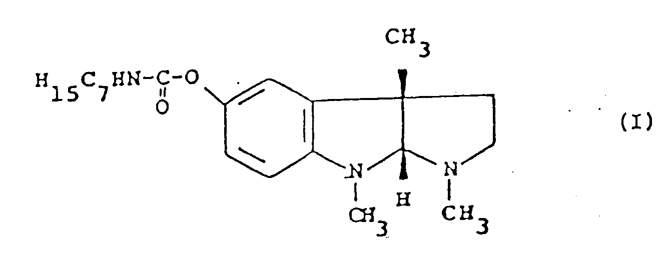 umfassend die Umsetzung von Eserolin (II) mit Heptylisocyanat, dadurch **gekennzeichnet,** daß man die Umsetzung in Gegenwart von a) katalytischen Mengen eines quaternären Ammoniumsalzes und b) katalytischen Mengen eines Metallcyanats in einem polaren aprotischen Lösungsmittel gemäß dem unten stehenden Reaktionsschema: 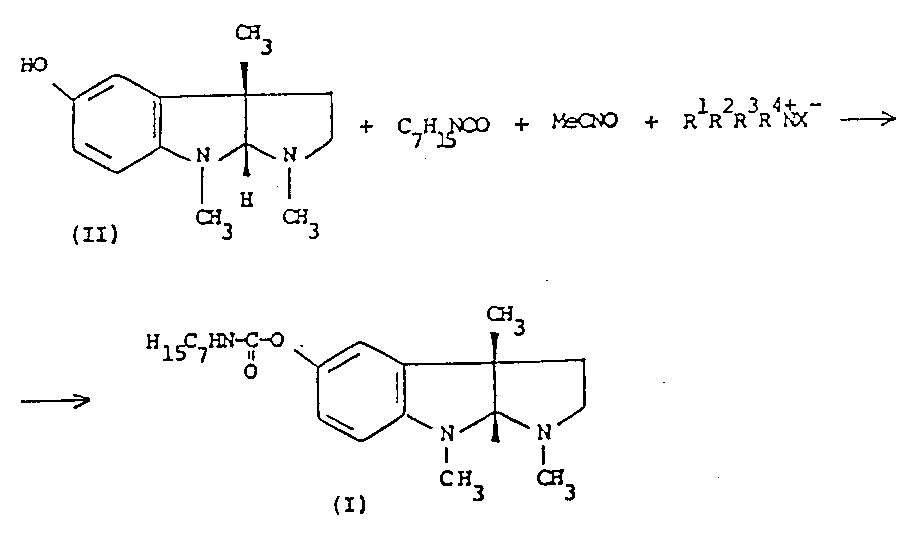 worin Me ein Alkalimetall oder das Äquivalent eines Erdalkalimetalls bedeutet; R₁-R₄, die gleich oder verschieden sein können, für C₁-C₁₂-Alkyl oder Aralkyl stehen; X ein Anion ist, durchführt.

2. Verfahren nach Anspruch 1, dadurch **gekennzeichnet,** daß das Molverhältnis von Eserolin zu Heptylisocyanat zwischen 1:1 und 1:5 liegt.

3. Verfahren nach Anspruch 1, dadurch **gekennzeichnet,** daß das Molverhältnis von Eserolin zu Heptylisocyanat zwischen 1:1 und 1:1,2 liegt.

4. Verfahren nach den Ansprüchen 1 bis 3, dadurch **gekennzeichnet,** daß das Molverhältnis von Eserolin zu Metallcyanat zwischen 1:0,5 und 1:0,01 liegt.

5. Verfahren nach den Ansprüchen 1 bis 3, dadurch **gekennzeichnet,** daß das Molverhältnis von Eserolin zu Metallcyanat zwischen 1:0,4 und 1:0,1 liegt.

6. Verfahren nach einem der vorstehenden Ansprüche, dadurch **gekennzeichnet,** daß das Molverhältnis von Eserolin zu quaternärem Ammoniumsalz zwischen 1:0,6 und 1:0,05 liegt.

7. Verfahren nach einem der vorstehenden Ansprüche, dadurch **gekennzeichnet,** daß das Molverhältnis von Eserolin zu quaternärem Ammoniumsalz zwischen 1:0,5 und 1:0,2 liegt.

8. Verfahren nach einem der vorstehenden Ansprüche, dadurch **gekennzeichnet,** daß man Acetonitril als Lösungsmittel verwendet.

9. Verfahren nach einem der vorstehenden Ansprüche, dadurch **gekennzeichnet,** daß man die Umsetzung bei Temperaturen zwischen 0°C und 40°C durchführt.

10. Verfahren nach einem der Ansprüche 1 bis 8, dadurch **gekennzeichnet,** daß man das Eserolin mit Heptylisocyanat, welches in situ ausgehend von einem 1-Halogenheptan und einem Metallcyanat in Gegenwart eines quaternären Ammoniumsalzes in polaren aprotischen Lösungsmitteln hergestellt wird, umsetzt.

11. Verfahren nach Anspruch 1, dadurch **gekennzeichnet,** daß Me Kalium ist, daß das C₁-C₁₂-Alkyl Ethyl oder n-Butyl ist, daß X Chlor oder Brom ist.

12. Verfahren nach Anspruch 10, dadurch **gekennzeichnet,** daß das 1-Halogenheptan 1-Bromheptan ist und daß das Metallcyanat Kaliumcyanat ist.

13. Verfahren nach Anspruch 10, dadurch **gekennzeichnet,** daß das polare aprotische Lösungsmittel Acetonitril ist.

## Revendications

1. Procédé pour préparer le 1,3a,8-triméthyl-1,2,3,3a,8,8a-hexahydropyrrole[2,3-b]indole-5(3aS, 8aR)-heptylcarbamate, de formule (I) : 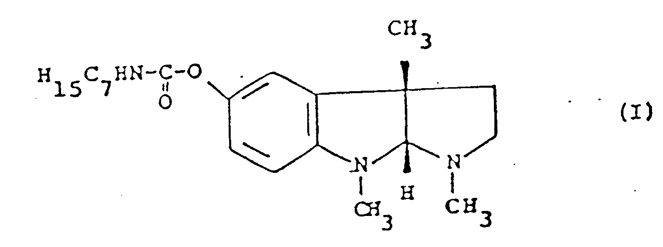 qui comprend la réaction de l'éséroline (II) avec de l'isocyanate d'heptyle, caractérisé en ce que cette réaction est mise en oeuvre en présence a) de quantités catalytiques d'un sel d'ammonium quaternaire et b) de quantités catalytiques d'un cyanate métallique, dans un solvant aprotique polaire, selon le schéma ci-après : 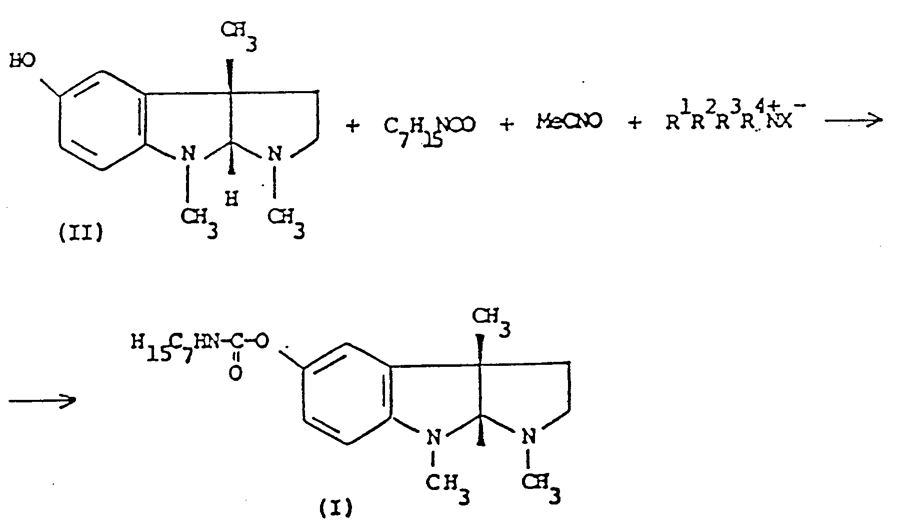 où Me est un métal alcalin ou l'équivalent d'un métal alcalino-terreux ; R₁ à R₄, qui peuvent être identiques ou différents, sont alkyle ou aralkyle en C₁ à C₁₂ ; X est un anion.

2. Procédé selon la revendication 1, caractérisé en ce que le rapport en moles de l'éséroline à l'isocyanate d'heptyle est compris entre 1:1 et 1:5.

3. Procédé selon la revendication 1, caractérisé en ce que le rapport en moles de l'éséroline à l'isocyanate d'heptyle est compris entre 1:1 et 1:1,2.

4. Procédé selon les revendications 1 à 3, caractérisé en ce que le rapport en moles de l'éséroline au cyanate métallique est compris entre 1:0,5 et 1:0,01.

5. Procédé selon les revendications 1 à 3, caractérisé en ce que le rapport en moles de l'éséroline au cyanate métallique est compris entre 1:0,4 et 1:0,1.

6. Procédé selon l'une quelconque des revendications précédentes, caractérisé en ce que le rapport en moles de l'éséroline au sel d'ammonium quaternaire est compris entre 1:0,6 et 1:0,05.

7. Procédé selon l'une quelconque des revendications précédentes, caractérisé en ce que le rapport en moles de l'éséroline au sel d'ammonium quaternaire est compris entre 1:0,5 et 1:0,2.

8. Procédé selon l'une quelconque des revendications précédentes, caractérisé en ce qu'on utilise l'acétonitrile comme solvant.

9. Procédé selon l'une quelconque des revendications précédentes, caractérisé en ce que la réaction est mise en oeuvre à des températures comprises entre 0 et 40°C.

10. Procédé selon l'une quelconque des revendications 1 à 8, caractérisé en ce qu'on fait réagir l'éséroline avec l'isocyanate d'heptyle, que l'on prépare in situ à partir d'un 1-halogénoheptane et d'un cyanate métallique en présence d'un sel d'ammonium quaternaire, dans des solvants aprotiques polaires.

11. Procédé selon la revendication 1, dans lequel Me est le potassium ; alkyle en C₁ à C₁₂ est éthyle ou n-butyle ; X est chlore ou brome.

12. Procédé selon la revendication 10, dans lequel le 1-halogénoheptane est le 1-bromoheptane, et le cyanate métallique est le cyanate de potassium.

13. Procédé selon la revendication 10, dans lequel le solvant aprotique polaire est l'acétonitrile.
